# EUROPÄISCHE PATENTSCHRIFT

(11) **EP 0 704 441 B1**
(45) Veröffentlichungstag und Bekanntmachung des Hinweises auf die Patenterteilung: **30.10.2002**
(21) Anmeldenummer: 95113232.3
(22) Anmeldetag: 23.08.1995
(51) Int. Cl.: C07D 317/12

(54) **Verfahren zur Herstellung von 2-Vinyl-1,3-dioxolan**
Process for the preparation of 2-vinyl-1,3-dioxolan
Procédé pour la préparation de 2-vinyl-1,3-dioxolane

(30) Priorität: 30.09.1994 DE 4435009
(43) Veröffentlichungstag der Anmeldung: 03.04.1996
(73) Patentinhaber: Degussa AG, 40474 Düsseldorf (DE)
(72) Erfinder: Höpp, Mathias, Dr., D-63599 Biebergemünd (DE); Arntz, Dietrich, Dr., D-61440 Oberursel (DE); Böck, Wolfgang, Dr., D-63505 Langenselbold (DE); Bosse-Plois, Andreas, D-53332 Bornheim (DE); Raible, Klaus, D-60388 Frankfurt (DE)

(56) Entgegenhaltungen:
- EP-A- 0 491 142
- US-A- 2 862 007
- US-A- 3 014 924
- US-A- 4 108 869
- JOURNAL OF ORGANIC CHEMISTRY, Bd. 25, 1960, Seiten 319-24, XP002017908 R.F. FISCHER ET AL.: "Cyclic Acrolein Acetals"
- JOURNAL FÜR PRAKTISCHE CHEMIE, Bd. 327, Nr. 4, 1985, Seiten 543-54, XP002017909 A. PIASECKI ET AL.: "Reaction Products of Prop-2-enal with 1,2- and 1,3-Diols"
- JOURNAL FÜR PRAKTISCHE CHEMIE, Bd. 329, Nr. 4, 1987, Seiten 579-86, XP002017910 A. PIASECKI: "Reaction Products of Prop-2-enal and But-2-enal with Mixture: n-Aliphatic Alcohol and Ethylene Glycol"
- RÖMPP CHEMIE-LEXIKON, 9. Auflage, Bd. 1 (1989), Seite 528
- RÖMPP CHEMIE-LEXIKON, 9. Auflage, Bd. 2 (1990), Seiten 1257-1258

## Beschreibung

Die Erfindung betrifft ein Verfahren zur Herstellung von 2-Vinyl-1,3-dioxolan. Das Verfahren basiert auf der säurekatalysierten Umsetzung von Acrolein mit Ethylenglykol und eignet sich insbesondere zur kontinuierlichen Herstellung von 2-Vinyl-1,3-dioxolan mit hoher Selektivität.

Es ist bekannt, cyclische Acetale des Acroleins durch Umsetzung von Acrolein mit dem entsprechenden Diol in Gegenwart eines festen oder gelösten sauren Katalysators herzustellen - US 3,014,924 und J. Org. Chem. (1960), Seiten 319-324.

Es ist aüs US 3.014.924 bekannt, 2-Vinyl-1,3-dioxolane, die in der 4 und 5 Position weitere Substituenten aufweisen, herzustellen.

Die Reaktionstemperatur kann dabei zwischen 50 und 150 °C liegen.

Im Verfahren gemäß US 3,014,924 werden als Katalysatoren mit Mineralsäuren belegte hochporöse Trägermaterialien, wie Kieselsäure, Kieselgel, Silicoaluminate, eingesetzt. Die Umsetzung erfolgt bei Temperaturen zwischen 50 und 150 °C, inbesondere 100 und 125 °C, wobei mittels eines organischen Lösungsmittels, wie beispielsweise Benzol, Toluol, Chloroform oder Cyclohexan, das Reaktionswasser azeotrop abdestilliert wird. Nachteilig an diesem Verfahren ist die geringe Raum-Zeit-Ausbeute. Die bei Verwendung einer geringen Katalysatormenge erforderliche hohe Reaktionstemperatur birgt in Verbindung mit der langen Reaktionsdauer die Gefahr der Nebenproduktbildung; eine erhöhte Katalysatormenge beschleunigt zwar die Acetalbildung, gleichzeitig werden aber Nebenprodukte in nicht tolerierbarer Menge gebildet (siehe Journal für praktische Chemie 327, 543 -54 (1985). Im Hinblick auf eine kontinuierliche Prozeßführung wird von der Verwendung von Acrolein als Azeotropschleppmittel wegen dadurch begünstigter Nebenproduktbildung abgeraten. Schließlich ist die Standzeit der Katalysatoren für ein technisches Verfahren zu gering, da die Mineralsäure vom Träger abgelöst wird.

Es ist aus der US 4,108,869 - dieses Dokument richtet sich ausschließlich auf die Umsetzung von 1,3-Diolen, nicht aber 1,2-Diolen, mit Acrolein - bekannt, Acrolein unter homogener Katalyse mit einem 1,3-Diol zum 2-Vinyl-1,3-dioxan umzusetzen, wobei Mineralsäuren oder Sulfonsäuren als Katalysator dienen: Das 1,3-Diol wird von oben in eine Extraktionssäule eingespeist und enthält die Säure. In der Mitte der Säule wird Acrolein eingespeist, welches mit dem nach unten fließenden Diol reagiert. Von unten wird im Gegenstrom ein Lösungsmittel eingespeist, welches sich mit dem 1,3-Diol schlecht mischt, z. B. Hexan. Die Hexan- und die wäßrige 1,3-Diol-Phase werden destillativ aufgearbeitet. Dieses Verfahren läßt sich nicht befriedigend auf die Herstellung von 2-Vinyl-1,3-dioxolan übertragen, da mit Ethylenglykol die Selektivität der Umsetzung geringer und damit der Nebenproduktanteil höher ist - mit Ethylenglykol tritt schon bei Raumtemperatur vermehrt Addition an die CC-Doppelbindung des Acroleins ein. Wird die Reaktion bei tieferer Temperatur durchgeführt, so bereitet zusätzlich die Abführung der Reaktionswärme bei dieser Reaktionsführung große Probleme.

In der EP-B 0 491 142 wird die Herstellung cyclischer Acroleinglycerinacetale durch Umsetzung von Acrolein mit Glycerin an stark sauren Ionentauschern beschrieben. Das erhaltene Reaktionsgemisch wird nach Einstellung des pH-Werts, vorzugsweise auf 5,5 bis 6,5, direkt destilliert. Diese Verfahrensvariante ist bei der Herstellung von 2-Vinyl-1,3-dioxolan (VDL) nachteilig, da wegen der ähnlichen Siedepunkte von Wasser und VDL auf der Kolonne eine Trennung Wasser/VDL erfolgen muß; bei erhöhter Temperatur erfolgt aber immer teilweise Rückspaltung zu Acrolein und Ethylenglykol.

Aufgabe der vorliegenden Erfindung ist, ein Verfahren zur Herstellung von 2-Vinyl-1,3-dioxolan zur Verfügung zu stellen, das die Nachteile des Standes der Technik überwindet. Das Verfahren sollte sich im technischen Maßstab kontinuierlich durchführen lassen und zu einer hohen Selektivität, bezogen auf Acrolein, und Produktreinheit führen. Schließlich sollte das Verfahren auch den Einsatz eines Rohacroleins, also eines mit Acetaldehyd verunreinigten Acroleins, erlauben.

Gefunden wurde ein Verfahren zur Herstellung von 2-Vinyl-1,3-dioxolan durch Umsetzung von Acrolein mit Ethylenglykol in Gegenwart eines festen sauren Katalysators und Aufarbeitung des vom Katalysator befreiten Reaktionsgemischs, das dadurch gekennzeichnet ist, daß man die Umsetzung bei einer Temperatur von unter 50 °C durchführt und das Reaktionsgemisch unter Verwendung eines Ethylenglykol im wesentlichen nicht lösenden organischen Lösungsmittels mit einem Siedepunkt oberhalb 130 °C extrahiert und die dabei erhaltene Ethylenglykol enthaltende wäßrige Phase und die 2-Vinyl-1,3-dioxolan enthaltende organische Phase destillativ aufarbeitet.

Die Umsetzung der Reaktionspartner erfolgt in an sich beliebiger Weise, etwa durch Mischen der Reaktionspartner und Kontaktieren des Gemischs mit dem sauren festen Katalysator oder durch Überleiten des Gemischs über einen sauren festen Katalysator.

Das Molverhältnis Acrolein zu Ethylenglykol ist für die Umsetzung wenig kritisch, und jede der beiden Ausgangskomponenten kann im Überschuß eingesetzt werden. Man verwendet im allgemeinen Molverhältnisse im Bereich 5:1 bis 1:5, vorzugsweise jedoch im Bereich 3:1 bis 1:3.

Das erfindungsgemäße Verfahren wird bei niedrigen Temperaturen durchgeführt, um die Nebenproduktbildung zu minimieren. Mit niedrigen Temperaturen sind Temperaturen unter 50 °C gemeint und insbesondere Temperaturen unter 15 °C.

Als Katalysator werden feste, im Reaktionsgemisch unlösliche saure Katalysatoren verwendet. Einsetzbar sind insbesondere anorganische und organische Ionentauscher in der sauren Form. Stark saure Ionentauscher werden bevorzugt. Unter den organischen Ionentauschern sind Austauscherharze auf der Basis von Styrol/Divinylbenzol-Copolymeren mit Sulfonat- oder Phosphonatgruppen, wobei solche mit stärker sauren Sulfonatgruppen bevorzugt werden, besonders geeignet. Auch marktübliche perfluorierte Sulfonsäureharze sind einsetzbar. Unter den anorganischen Ionenaustauschern sind saure oder starksaure Zeolithe, wie solche vom Typ H-ZSM, hervorzuheben.

Das erfindungsgemäße Verfahren läßt sich diskontinuierlich, beispielsweise in einem Rührreaktor, oder kontinuierlich, beispielsweise in einem Schlaufenreaktor, wobei der Katalysator als Festbett oder Fließbett angeordnet sein kann, durchführen. Prinzipiell sind alle Reaktorformen geeignet, welche einen ausreichenden Kontakt zwischen dem Reaktionsgemisch und dem festen Katalysator gewährleisten. Bei kontinuierlicher Verfahrensweise werden vorzugsweise Acrolein und Ethylenglykol im gewünschten Molverhältnis kontinuierlich zu einem Acrolein, Ethylenglykol, 2-Vinyl-1,3-dioxolan und Wasser enthaltenden Reaktionsgemisch, vorzugsweise einem Gleichgewichtsreaktionsgemisch, eingespeist und die entsprechende Menge Reaktionsgemisch nach Passage durch das Katalysatorbett zur Aufarbeitung abgezogen. Für die Kontaktzeit des Reaktionsgemischs, ausgedrückt durch den LHSV-Wert (liquid hourly space velocity), gilt, daß der LHSV-Wert zwischen 1 und 30 h⁻¹, bevorzugt zwischen 3 und 15 h⁻¹, liegen sollte.

Nach der Umsetzung besteht das Reaktionsgemisch im wesentlichen aus 2-Vinyl-1,3-dioxolan sowie den Reaktionspartnern Acrolein und Ethylenglykol und dem Reaktionswasser. Unter dem Begriff "im wesentlichen" wird verstanden, daß das Reaktionsgemisch zusätzlich Nebenprodukte enthält, hauptsächlich das Addukt von Ethylenglykol an 2-Vinyl-1,3-dioxolan, das 2-(5'-Hydroxy-3'-oxopentyl)-1,3-dioxolan. Die Zusammensetzung des Reaktionsgemischs richtet sich nach dem gewählten Molverhältnis Acrolein zu Ethylenglykol und der Reaktionstemperatur und entspricht vorzugsweise der jeweiligen Gleichgewichtszusammensetzung.

Nach der Umsetzung wird, sofern erforderlich, das vom Katalysator befreite Reaktionsgemisch unter Verwendung eines basischen Stoffes, etwa eines Trialkanolamins, auf einen pH-Wert von 4,5 bis 7,0, vorzugsweise von 5,5 bis 6,5, eingestellt, bestimmt nach zehnfacher Verdünnung mit Wasser, und dann mit einem unpolaren bis wenig polaren organischen Lösungsmittels extrahiert. Die Teilneutralisation ist selten notwendig. Das Lösungsmittel soll Ethylenglykol im wesentlichen nicht lösen, und der Siedepunkt des Lösungsmittels soll oberhalb 130 °C, vorzugsweise oberhalb 150 °C, liegen. Geeignete organische Lösungsmittel sind aliphatische lineare oder verzweigte Kohlenwasserstoffe, etwa n-C₁₀- bis C₁₂-Alkane oder sogenannte Isoparaffine mit einem Siedepunkt im Bereich von 170 bis 250 °C; auch cycloaliphatische Kohlenwasserstoffe, wie Decalin, sowie ein- oder mehrfach alkylsubstituierte aromatische Kohlenwasserstoffe, wie Di-, Tri- und Tetramethylbenzol, sind geeignete Extraktionsmittel. Üblicherweise werden 0,3 bis 3 Volumteile organisches Lösungsmittel pro Volumteil Reaktionsgemisch eingesetzt. Bei der Extraktion bilden sich eine Wasser und Ethylenglykol sowie hochsiedende Nebenprodukte enthaltende Phase und eine 2-Vinyl-1,3-dioxolan, Acrolein und organisches Lösungsmittel enthaltende Phase, welche in bekannter Weise voneinander getrennt werden.

Bei Bedarf kann es zweckmäßig sein, die wäßrige und/oder die 2-Vinyl-1,3-dioxolan enthaltende organische Phase zwecks ausreichender Abtrennung gegebenenfalls noch dispergierter sehr feiner Tröpfchen der anderen Phase und damit Gewinnung sehr reiner Phasen über Coalescer zu leiten. Besonders reine Phasen werden mittels Coalescern erhalten, wenn der zu reinigenden Phase vor der Passage durch den Coalescer 1 bis 10 Volumen-% der bereits dipergierten Phase zugemischt werden, also Wasser zur organischen Phase und organisches Lösungsmittel zur wäßrigen Phase.

Im Falle der Verwendung einer Extraktionskolonne wird diese zweckmäßigerweise so betrieben, daß das Reaktionsgemisch in den oberen Teil eingespeist wird. Zur besseren Phasenseparation werden auf den Kopf der Extraktionskolonne oder unmittelbar in das zu extrahierende Reaktionsgemisch 0,01 bis 1 Volumenteile Wasser pro Volumenteil Reaktionsgemisch aufgegeben. Am Fuß der Extraktionskolonne werden 0,3 bis 3 Volumenteile Extraktionsmittel pro Volumenteil Reaktionsgemisch zudosiert.

Nach der Extraktion und Phasentrennung wird aus der organischen Phase zunächst Acrolein abdestilliert, dann das Produkt; das hochsiedende Extraktionsmittel wird bei kontinuierlicher Betriebsweise direkt, also ohne Destillation, in die Extraktion eingespeist. Aus der wäßrigen Phase wird zunächst Acrolein abdestilliert, dann das Reaktionswasser und zuletzt das Ethylenglykol. Die hochsiedenden Nebenprodukte und das Reaktionswasser werden ausgeschleust; die aus beiden Phasen zurückgewonnenen nicht umgesetzten Edukte werden in die Reaktionsstufe zurückgeführt.

Ein Vorteil der Erfindung beruht auf der Möglichkeit, ein sogenanntes Roh-Acrolein für die Reaktion zu verwenden. Dieses Roh-Acrolein enthält neben den üblichen 3,5 % Wasser noch 2 % Acetaldehyd. Die Verunreinigung Acetaldehyd wird während der Reaktion quantitativ in das entsprechende Acetal 2-Methyl-1,3-dioxolan überführt. Bei der Extraktion gelangt diese Verbindung in die organische Phase und muß zusätzlich destillativ abgetrennt werden. Die organische Phase wird hier so aufgearbeitet, daß zunächst das Acrolein, dann das 2-Methyl-1,3-dioxolan und zum Schluß das 2-Vinyl-1,3-dioxolan abdestilliert werden.

Das erfindungsgemäße Verfahren zeichnet sich dadurch aus, daß das 2-Vinyl-1,3-dioxolan in überraschend hoher Selektivität, nämlich um und teilweise über 90 %, bezogen auf Acrolein, gewonnen werden kann. Gemäß J. Prakt. Chem. 329 (4), 579-586 (1987) resultiert aus der Umsetzung von Ethylenglykol mit Acrolein nämlich ein komplexes Reaktionsgemisch, dessen Hauptprodukt 2-(5'-Hydroxy-3'-oxopentyl)-1,3-dioxolan ist. Das Verfahren läßt sich im technischen Maßstab mit hoher Katalysatorstandzeit ausführen. Nicht umgesetzte Edukte lassen sich einfach zurückgewinnen und einem Folgeansatz zuführen. Das für die Extraktion verwendete Lösungsmittel kann, ohne daß es destilliert werden muß, rezykliert werden.

### Beispiel 1

In einem Schlaufenreaktor mit 40 l Gesamtvolume ist ein Katalysatorfestbett mit 2 l eines stark sauren Ionentauschers (K 2431 der Fa. Bayer) eingebaut. Ein Reaktionsgemisch mit der ungefähren Zusammensetzung 34 Gew.-% Acrolein, 36 Gew.-% Ethylenglykol, 23 Gew.-% 2-Vinyl-1,3-dioxolan, 4 Gew.-% Wasser und 3 Gew.-% Nebenprodukte wird fortlaufend bei einer Innentemperatur von 5 °C im Kreis gepumpt. Kontinuierlich werden 4,6 kg Acrolein/h und 5,0 kg Ethylenglykol/h eingespeist und die entsprechende Menge Reaktionsgemisch (9,6 kg) abgezogen (LHSV = 4,8). Das abfließende Reaktionsgemisch wird am Kopf der Extraktionskolonne zusammen mit 1,0 kg Wasser/h eingespeist. Im Gegenstrom werden am Fuß der Extraktionskolonne 7,3 l/h Dekan eingespeist. Die abfließende organische und wäßrige Phase werden einer fraktionierten Destillation unterworfen, wobei von der organischen Phase zunächst Acrolein und dann das Produkt abdestilliert werden. Aus der wäßrigen Phase wird ebenfalls zunächst Acrolein abdestilliert, dann Wasser und dann Ethylenglykol. Man erhält stündlich 3,3 kg Acrolein, 3,5 kg Ethylenglykol, 2,1 kg 2-Vinyl-1,3-dioxolan, 0,4 kg Wasser und 0,3 kg Hochsieder.

### Beispiel 2

Wie Beispiel 1, jedoch setzt sich das eingespeiste Acrolein aus 1,6 kg Roh-Acrolein mit 94,5 Gew.-% Acrolein, 3,5 Gew.-% Wasser und 2 Gew-% Acetaldehyd sowie 3,0 kg rezykliertem Acrolein (96 % Acrolein, 4 % Wasser) zusammen.

Das erhaltene Reaktionsgemisch hat die Zusammensetzung 31 Gew.-% Acrolein, 34 Gew.-% Ethylenglykol, 23 Gew.-% 2-Vinyl-1,3-dioxolan, 1,6 Gew.-% 2-Methyl-1,3-dioxolan, 4 Gew.-% Wasser und 3 Gew.-% Nebenprodukte.

Nach destillativer Aufarbeitung erhält man stündlich 3,2 kg Acrolein, 3,4 kg Ethylenglykol, 2,1 kg 2-Vinyl-1,3-dioxolan, 0,15 kg 2-Methyl-1,3-dioxolan, 0,4 kg Wasser und 0,3 kg Hochsieder. Das zurückgewonnene Acrolein und Ethylenglykol werden erneut eingesetzt. Das eingesetzte Acrolein wird zu 30,1 % umgesetzt mit einer Selektivität von 89,1 %, das eingesetzte Ethylenglykol wird zu 32,0 % umgesetzt mit einer Selektivität von 81,3 %.

### Vergleichsbeispiel

### Herstellung von VDL gemäß US 3,014,924

372 g Ethylenglykol, 750 ml Benzol, 2,7 g Katalysator (0,5 % H₂SO₄ auf Kieselgel) und 120 g Acrolein werden in einer Apparatur mit Azeotropabschneider vorgelegt. Anschließend wird unter Rühren zum Sieden erhitzt und das abgeschleppte Wasser wird abgetrennt. Über 4 Stunden werden weitere 250 g Acrolein so zudosiert, daß die Innentemperatur ziwschen 71 und 74 °C bleibt. Anschließend wird weiter erhitzt, bis die Innentemperatur 86 °C erreicht. Die Gesamtreaktionsdauer beträgt 12 h, insgesamt werden 123 ml Wasser abgeschieden. Nach dem Abkühlen wird das Reaktionsgemisch filtriert und fraktioniert destilliert. Man erhält 533 g 2-Vinyl-1,3-dioxolan (89 % Ausbeute).

## Patentansprüche

1. Verfahren zur kontinuierlichen Herstellung von 2-Vinyl-1,3-dioxolan durch Umsetzung von Acrolein mit Ethylenglykol in Gegenwart eines festen sauren Katalysators, **dadurch gekennzeichnet, daß** man
a) Acrolein und Ethylenglykol in ein Gleichgewichtsreaktionsgemisch, enthaltend 2-Vinyl-1,3-dioxolan Ethylenglykol, Acrolein und Wasser, eindosiert,
b) das Gleichgewichtsreaktionsgemisch über einen als sauren Festbettkatalysator vorliegenden organischen oder anorganischen Ionenaustauscher leitet,
c) die Umsetzung bei einer Temperatur von unter 50°C durchführt,
d) danach eine den zudosierten Stoffen äquivalente Menge an Reaktionsgemisch der Aufarbeitung zuführt,
e) diese unter Verwendung eines Ethylenglykol im wesentlichen nicht lösenden organischen Lösungsmittels mit einem Siedepunkt oberhalb 130°C extrahiert,
f) die dabei erhaltene Ethylenglykol enthaltende wässrige Phase und die 2-Vinyl-1,3-dioxolan enthaltende organische Phase destillativ aufarbeitet und
g) die verbleibende Menge Reaktionsgemisch rezykliert.

2. Verfahren nach Anspruch,
**dadurch gekennzeichnet,**
**daß** man die Extraktion unter Verwendung mindestens einer Extraktionskolonne durchführt, wobei dem in den oberen Teil der Kolonne eingespeisten Reaktionsgemisch oder dem Kolonnenkopf 0,01 bis 1 Volumenteile Wasser pro Volumenteil Reaktionsgemisch zudosiert werden.

3. Verfahren nach einem oder mehreren der Ansprüche 1 bis 2,
**dadurch gekennzeichnet,**
**daß** man das vom Katalysator befreite Reaktionsgemisch vor der Extraktion mit einer solchen Menge eines pH-Wert erhöhenden Stoffes versetzt, daß der pH-Wert, gemesssen in zehnfacher Verdünnung mit Wasser, im Bereich zwischen 4,5 und 7,0, vorzugsweise zwischen 5,5 und 6,5, liegt.

4. Verfahren nach einem oder mehreren der Ansprüche 1 bis 3,
**dadurch gekennzeichnet,**
**daß** man die Umsetzung bei 0 bis 15 °C durchführt.

5. Verfahren nach einem oder mehreren der Ansprüche 1 bis 4,
**dadurch gekennzeichnet,**
**daß** man zur Extraktion ein Lösungsmittel mit einem Siedepunkt oberhalb 150 °C verwendet, vorzugsweise einen aliphatischen linaren oder verzweigten Kohlenwasserstoff, einen cycloaliphatischen Kohlenwasserstoff oder einen ein- oder mehrfach alkylsubstituierten aromatischen Kohlenwasserstoff.

## Claims

1. Process for the continuous production of 2-vinyl-1,3-dioxolane by reacting acrolein with ethylene glycol in the presence of a solid acidic catalyst, **characterised in that**
a) acrolein and ethylene glycol are metered into an equilibrium reaction mixture containing 2-vinyl-1,3-dioxolane ethylene glycol [sic], acrolein and water,
b) the equilibrium reaction mixture is passed through an organic or inorganic ion exchanger in the form of an acidic fixed bed catalyst,
c) the reaction is carried out at a temperature of less than 50°C,
d) a quantity of reaction mixture equivalent to the substances metered in is then fed to the work-up,
e) this is extracted using an organic solvent in which ethylene glycol is substantially insoluble, with a boiling point of more than 130°C,
f) the aqueous phase containing ethylene glycol thus obtained and the organic phase containing 2-vinyl-1,3-dioxolane are worked up by distillation and
g) the remaining quantity of reaction mixture is recycled.

2. Process according to claim [sic],
**characterised in that**
the extraction is performed using at least one extraction column, with 0.01 to 1 part by volume of water per part by volume of reaction mixture being metered into the reaction mixture fed into the upper part of the column or the column head.

3. Process according to one or more of claims 1 to 2,
**characterised in that**
a quantity of a pH-increasing substance such that the pH, measured in a tenfold dilution in water, is in the range of between 4.5 and 7.0, preferably between 5.5 and 6.5, is added to the reaction mixture freed of the catalyst.

4. Process according to one or more of claims 1 to 3,
**characterised in that**
the reaction is carried out at 0 to 15°C.

5. Process according to one or more of claims 1 to 4,
**characterised in that**
a solvent with a boiling point of more than 150°C is used for the extraction, preferably an aliphatic, linar [sic] or branched hydrocarbon, a cycloaliphatic hydrocarbon or a mono- or polyalkyl substituted aromatic hydrocarbon.

## Revendications

1. Procédé de production en continu du 2-vinyl-1,3-dioxolane par réaction de l'aeroline avec l'éthylène glycol en présence d'un catalyseur solide acide,
**caractérisé en ce qu'**on
a) admet par doses de l'acroléine et de l'éthylène glycol dans un mélange réactionnel équilibré contenant du 2-vinyl-1,3-dioxolane, de l'éthylène glycol, de l'acroléine et de l'eau ;
b) on conduit le mélange réactionnel équilibré sur un échangeur d'ions organique ou minéral présent, comme catalyseur en lit fixe acide ;
c) on effectue la réaction à une température inférieure à 50°C ;
d) ensuite on amène une quantité équivalente aux substances ajoutées par doses, en mélange réactionnel, à la purification,
e) on extrait celui-ci en utilisant un solvant organique qui ne dissout pas essentiellement l'éthylène glycol, ayant un point d'ébullition au dessus de 130°C ;
f) on purifie la phase aqueuse contenant l'éthylène glycol obtenu et la phase organique contenant le 2-vinyl-1,3-dioxolane par distillation ; et
g) on recycle la quantité restante de mélange réactionnel.

2. Procédé selon la revendication 1,
**caractérisé en ce qu'**
on effectue l'extraction en utilisant au moins une colonne d'extraction, dans laquelle on admet par doses au mélange réactionnel injecté à la partie supérieure de la colonne ou à la tête de la colonne, de 0.01 à 1 partie en volume d'eau par partie en volume de mélange réactionnel.

3. Procédé selon l'une ou plusieurs des revendications 1 à 2,
**caractérisé en ce qu'**
on mélange le mélange réactionnel débarrassé du catalyseur avant l'extraction avec une quantité telle d'une substance qui élève la valeur du pH que la valeur du pH mesuré en dilution de dix fois avec de l'eau, se situe dans la zone comprise entre 4,5 et 7,0 de préférence entre 5,5 et 6,5.

4. Procédé selon l'une quelconque des revendications 1 à 3,
**caractérisé en ce que**
la réaction est effectuée entre 0 et 15°C.

5. Procédé selon l'une ou plusieurs des revendications 1 à 4,
**caractérisé en ce qu'**
on utilise pour l'extraction un solvant ayant un point d'ébullition au dessus de 150°C, de préférence un hydrocarbure aliphatique linéaire ou ramifié, un hydrocarbure cycloaliphatique ou une hydrocarbure aromatique substitué une fois ou plusieurs fols par un alkyle.
